Europäisches Patentamt

European Patent Office

Office européen des brevets

(11) Veröffentlichungsnummer: **0 302 336 B1**

# (12) EUROPÄISCHE PATENTSCHRIFT

(45) Veröffentlichungstag der Patentschrift: **04.03.92**

(21) Anmeldenummer: **88111930.9**

(22) Anmeldetag: **25.07.88**

(51) Int. Cl.⁵: **C07C 1/20**, C07C 29/00

(54) Verfahren zur Spaltung von Alkyl-tert.-alkylethern.

(30) Priorität: **04.08.87 DE 3725850**
**11.05.88 DE 3816121**

(43) Veröffentlichungstag der Anmeldung:
**08.02.89 Patentblatt 89/06**

(45) Bekanntmachung des Hinweises auf die
Patenterteilung:
**04.03.92 Patentblatt 92/10**

(84) Benannte Vertragsstaaten:
**DE FR GB IT NL**

(56) Entgegenhaltungen:
**EP-A- 0 008 860**
**DE-A- 3 210 435**

(73) Patentinhaber: **EC ERDÖLCHEMIE GMBH**
**Postfach 75 20 02**
**W-5000 Köln 71(DE)**

(72) Erfinder: **Gabel, Christian, Dr.**
**Goethestrasse 69**
**W-4047 Dormagen 1(DE)**
Erfinder: **Schleppinghoff, Bernhard, Dr.**
**Adolf-Kolping-Strasse 5**
**W-4047 Dormagen 1(DE)**
Erfinder: **Scheef, Hans-Volker**
**Goethestrasse 69**
**W-4047 Dormagen 1(DE)**
Erfinder: **Köhler, Hans-Dieter, Dr.**
**Stürzelbergerstrasse 71**
**W-4047 Dormagen 5(DE)**

(74) Vertreter: **Zobel, Manfred, Dr. et al**
**c/o BAYER AG Konzernverwaltung RP Paten-**
**te Konzern**
**W-5090 Leverkusen 1 Bayerwerk(DE)**

**Beschreibung**

Die vorliegende Erfindung betrifft ein Verfahren zur Spaltung von Alkyl-tert.-alkylethern in die zugrundeliegenden Alkanole und tert.-Olefine in einer Kolonnenapparatur.

Es ist bereits bekannt (DE-OS 32 10 435), Methyl-tert.-butylether (MTBE) in einer Kolonnenapparatur in Methanol und i-Buten zu spalten, wobei als Katalysator ein Kationenaustauscher auf der Basis von sulfonierten Styrol-Divinylbenzol-Harzen in verschiedenen Kolonnenschüssen angeordnet wird, zwischen denen jeweils Kolonnenschüsse mit 6 Destillationsböden jedoch ohne Kationenaustauscher angeordnet sind. Das MTBE wird oberhalb der obersten Katalysatorschüttung eingespeist. Die Katalysatorschicht wird von dem eingespeisten flüssigen MTBE und dem während der Reaktion entstehenden Methanol von oben nach unten durchströmt und vom gasförmigen entstehenden i-Buten, gegebenenfalls als Azeotrop mit Methanol, von unten nach oben durchströmt. Für die Reaktionsbedingungen findet sich in der obigen DE-OS eine Angabe von 5 bar und einer Kolonnentemperatur zwischen 45°C (Kopftemperatur) und 100°C (Sumpftemperatur). In einem solchen Verfahren ist es zu erwarten, daß 1. die Kolonne durch die dichte Katalysatorpackung sehr leicht staut und damit maßgeblich an Trennleistung einbüßt und 2. die Katalysatorschüttung durch die entgegengesetzt durchlaufenden flüssigen bzw. gasförmigen Ströme ständig umgeschichtet wird, wobei eine Kanalbildung in den Katalysatorschüttungen sehr wahrscheinlich ist. Durch solch eine Kanalbildung würde der größte Teil der Katalysatormasse von den durchströmenden Stoffen nicht berührt und damit völlig ungenügend ausgenutzt. Die Montage einer solchen Kolonnenapparatur, die Einbringung des Katalysators in den vorgesehenen Kolonnenschüssen und natürlich jeder Austausch des Katalysators gestalten sich umständlich und damit kostspielig. i-Buten, das erst in den unteren Katalysatorschichten entstanden ist, muß auf dem Wege nach oben sämtliche darüberliegenden Katalysatorschichten und -schüttungen durchströmen; hierbei kann eine unerwünschte Dimerisierung zu Diisobutylen nicht ausgeschlossen werden, die die Ausbeute an i-Buten mindert und zusätzliche Aufarbeitungsschwierigkeiten für das Sumpfprodukt bringt. In ähnlicher Weise unterliegt Methanol, das durch Spaltung in den oberen Katalysatorschichten entstanden ist, bei seinem Wege zum Kolonnensumpf der Gefahr der Dimethyletherbildung an dem sauren Katalysatorharz. Die Bildung unerwünschter Nebenprodukte wird durch die hohe Temperatur im unteren Teil der Kolonne noch gefördert.

Auch im Verfahren nach EP 8860 wird eine mit einer katalytisch wirkenden Packung versehene Kolonne benutzt, die zur Spaltung von MTBE eingesetzt wird. Das MTBE wird im mittleren Bereich der Kolonne eingespeist. Auch hier müssen die Spaltprodukte Methanol und i-Buten durch große Bereiche der katalytisch wirkenden Packung wandern, wobei sie den oben geschilderten unerwünschten Umwandlungen unterliegen.

Es wurde ein Verfahren zur Spaltung von Alkyl-tert.-alkylethern in die zugrundeliegenden Alkanole und tert.-Olefine in Gegenwart von stark sauren Katalysatoren in einer Kolonnenapparatur gefunden, das dadurch gekennzeichnet ist, daß der stark saure Katalysator unlöslich und hochpolymer, sowie anorganisch oder organisch ist und im Umlauf der Sumpfheizung der Kolonne bereitgestellt wird.

Als zugrundeliegende Alkanole seien beispielsweise primäre oder sekundäre $C_1$-$C_4$-Alkanole genannt, wie Methanol, Ethanol, Propanol, i-Propanol, n-Butanol oder sec.-Butanol. In bevorzugter Weise seien die primären Alkanole genannt und unter diesen wieder Methanol oder Ethanol; Methanol ist besonders bevorzugt.

Als zugrundeliegende tert.-Olefine seien beispielsweise $C_4$-$C_7$-tert.-Olefine genannt, wie i-Buten, i-Amylene, i-Hexene oder i-Heptene. In bevorzugter Weise seien i-Buten und die i-Amylene genannt.

Als Alkyl-tert.-alkylether kommen daher in Frage: Methyl-tert.-butylether (MTBE), tert.-Amyl-methylether (TAME), Methyl-tert.-hexylether, Methyl-tert.-heptylether, Ethyl-tert.-butylether, Ethyl-tert.-amylether, Ethyl-tert.-hexylether, Ethyl-tert.-heptylether und andere.

Als stark saure Katalysatoren kommen unlösliche, hochpolymere Stoffe anorganischer oder organischer Natur in Frage. Hierzu gehören Stoffe wie saures $SiO_2$, saures $Al_2O_3$, saure Zeolithe, wie Mordenite und andere, aber auch neutrale Stoffe, die mit starken Säuren behandelt worden sind, wie mit $H_2SO_4$ oder $H_3PO_4$ getränkte Kieselsäuren, Aluminiumoxide oder andere anorganische Trägermaterialien, sowie sulfonierte Kohlen, ferner organische, stark saure Kationenaustauscher, wobei alle genannten Stoffe in der $H^+$-Form verstanden werden; sie bewirken eine heterogen-katalytische Spaltung der genannten Ether.

Von den genannten Katalysatoren werden die stark sauren Kationenaustauscher in der $H^+$-Form bevorzugt.

Als stark saure Kationenaustauscher kommen beispielsweise solche auf der Basis von Styrol-Divinylbenzol-Harzen, Phenol-Formaldehyd-Harzen oder Cumaron-Inden-Harzen in Frage, deren aromatische Kerne Sulfonsäuregruppen tragen. In bevorzugter Weise kommen die unter verschiedenen Handelsnamen erhältlichen sulfonierten Styrol-Divinylbenzol-Harze in Frage. Diese Kationenaustauscher werden in der

$H^+$-Form eingesetzt.

Als Kolonnenapparatur kommt eine solche in Frage, wie sie dem Fachmann für Destillations- und/oder Extraktionszwecke bekannt ist und entsprechend ausgerüstet ist.

Erfindungsgemäß wird der stark saure Katalysator im Umlauf der Sumpfheizung der Kolonne bereitgestellt.

Unlösliche, polymere Katalysatoren der genannten Art zeigen keine Verluste über einen Reinigungsstrom. Dies gilt in besonderem Maße für stark saure Kationenaustauscher, die daher in der Folge beispielhaft für die weitere Beschreibung des erfindungsgemäßen Verfahrens stehen.

Der Sumpfumlauf durchströmt also erfindungsgemäß stets abwechselnd den Kationenaustauscher als Beispiel für einen unlöslichen, polymeren, stark sauren Katalysator und die indirekte Kolonnenheizung, die elektrisch oder mit Hilfe eines separaten Wäremträgerstroms (Dampf, Wärmeträgerflüssigkeit) betrieben wird. Der Sumpfumlauf kann hierbei durch die von der Sumpfheizung hervorgerufenen Konvektion oder als Zwangsumlauf mit Hilfe von Pumpen bewirkt werden. Hierbei kann der durch eine Pumpe bewirkte Zwangsumlauf auch entgegen der Konvektionswirkung geführt werden, so daß es grundsätzlich möglich ist, den Sumpfumlauf unterhalb seiner Entnahme in die Kolonne wieder zurückzuführen und damit den im Sumpf angeordneten Kationenaustauscher von unten her anzuströmen. Es ist jedoch bevorzugt, den Kationenaustauscher von oben her anzuströmen, was durch Konvektion oder durch Zwangsumlauf bewirkt werden kann.

In überraschend vorteilhafter Weise erlaubt die erfindungsgemäße Anordnung des Kationenaustauschers als Beispiel für einen unlöslichen, polymeren, stark sauren Katalysator die rasche Entfernung der Spaltprodukte vom Kationenaustauscher, so daß nachträgliche Reaktionen des tert.-Olefins (zum Dimeren) oder des Alkanols (zum Dialkylether) wirkungsvoll unterdrückt werden können. Insbesondere wird bei der Spaltung von Methyl-tert.-alkylethern die Bildung des Dimethylethers unterdrückt, der die Reindarstellung der Spaltprouldkte sehr erschwert. Desweiteren ist es durch die unmittelbare Nachbarschaft des angeordneten Kationenaustauschers und der Sumpfheizung möglich, die zur Spaltung der Alkyl-tert.-alkylether erforderliche Temperatur sehr viel genauer einzustellen und damit eine unnötige thermische Belastung der Reaktionsprodukte zu vermeiden.

Der Sumpfumlauf hat eine Temperatur von beispielsweise 50-100°C, bevorzugt 55-85°C, besonders bevorzugt 60-80°C. Für den Fall, daß Alkyl-tert.-alkylether mit mehr als 6 Gesamt-C-Atomen gespalten werden, kann es nötig sein, die Obergrenze der genannten Temperaturbereiche um 10 bis 20°C heraufzusetzen. Es kann weiterhin bei Benutzung der obengenannten anorganischen unlöslichen stark sauren Katalysatoren günstig sein, Temperaturen bis zu 200°C, bevorzugt bis zu 160°C, anzuwenden. Für den Fall der leichter siedenden Ether unter den obengenannten wird dann in fachmännischer Weise unter einem solchen Druck gearbeitet, daß am Fuß der Kolonne eine flüssige Phase aufrechterhalten bleibt.

Es ist bevorzugt, innerhalb des genannten Temperaturbereiches, sei es der Bereich von 50-100°C oder ein bei höheren Ethern um 10-20°C nach oben erweiterter Bereich oder schließlich der beschriebene, bis zu 200°C reichende Bereich bei der maximalen Siedetemperatur des im Sumpf umlaufenden Reaktionsgemisches zu arbeiten, die sich aufgrund der übrigen Kolonnenbedingungen einstellt. Hierdurch ist ein möglichst rasches Ausgasen der nach oben abströmenden gasförmigen Spaltprodukte gewährleistet. Unter den zu berücksichtigenden Kolonnenbedingungen sei besonders auf den einzustellenden Druck hingewiesen. Das erfindungsgemäße Verfahren kann grundsätzlich auch unter vermindertem Druck durchgeführt werden, jedoch ist ein solch verminderter Druck wegen der schwierigen Kondensation des tert.-Olefins am Kolonnenkopf weniger bevorzugt und findet nur bei tertiären Olefinen Anwendung, die mehr als 6 C-Atome aufweisen. Die Kolonne wird daher bevorzugt bei normalem oder schwach erhöhtem Druck, beispielsweise 1-5 bar, betrieben. Beim Arbeiten unter Normaldruck lastet jedoch auf dem Kolonnensumpf zumindest der dem Fachmann bekannte und von der Kolonnenhöhe abhängige Differenzdruck.

Die erfindungsgemäße Anordnung des Kationenaustauschers erlaubt Anströmgeschwindigkeiten durch den Sumpfumlauf in einen sehr großen Bereich. Diese Anströmgeschwindigkeiten werden definiert als LHSV (Liquid Hourly Space Velocity) und nehmen Werte von a = 1-100, bevorzugt 10-50 Liter Sumpfumlauf pro Liter Kationenaustauscher pro Stunde an. Der zu spaltende Ether wird dem Sumpfumlauf in einer Menge von 0,5-10, bevorzugt 3-8 l Ether pro l Kationenaustauscher pro Stunde zugesetzt.

Dem erfindungsgemäßen Verfahren wird das durch die Spaltung entstehende tert.-Olefin am Kopf der Kolonne, gegebenenfalls als Azeotrop mit gleichzeitig entstehendem Alkanol, entnommen. Der größte Teil des durch die Spaltung entstehenden Alkanols wird dem Sumpfablauf, gemeinsam mit geringen Mengen nicht gespalten Ethers und geringen Mengen an Nebenprodukten entnommen.

In einer vorteilhaften Variante des erfindungsgemäßen Verfahrens wird der Kolonne unterhalb der oberen Kolonnenböden ein Wasserstrom zugeführt. Dieser Wasserstrom bewirkt eine Wäsche des am Kolonnenkopf entnommenen tert.-Olefins und erlaubt die Entnahme des bei der Spaltung entstehenden Alkanols als Gemisch mit Wasser von einem der unteren Böden oberhalb des Kolonnensumpfes. Der Zulauf des zu spaltenden Alkyl-tert.-alkylethers liegt vorzugsweise unter der Entnahme des genannten Wasserstromes. Die Wassermenge beträgt hierbei 2-6 Gew.-Teile pro 1 Gew.-Teil der auszuwaschendem Alkanolmenge.

Bei dieser vorteilhaften Verfahrensvariante wird dem Sumpfablauf nur ein kleiner Reinigungsstrom entnommen, der im wesentlichen aus nicht umgesetztem Alkyl-tert.-alkylether und geringen Mengen an Hochsiedern besteht.

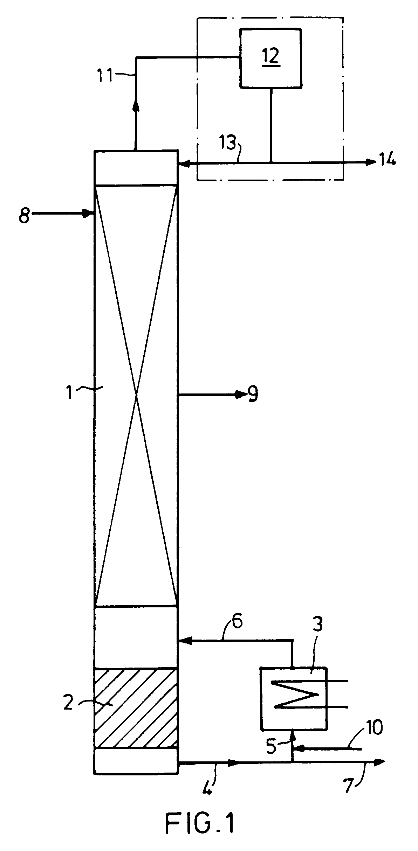

Anhand der beigefügten Abbildung sei das erfindungsgemäße Verfahren in seiner geschilderten günstigen Variante unter Benutzung eines unlöslichen, hochpolymeren, stark sauren Katalysators erläutert:

Eine mit Destillations- und/oder Extraktionseinrichtungen versehene Kolonne (1) wurde im Sumpfumlauf mit einer Schüttung von unlöslichem, hochpolymeren, stark saurem Katalysator (2) versehen. Zum Sumpfumlauf gehört ferner die indirekte Sumpfheizung (3), die in der Abbildung beispielhaft zeigt, daß der Sumpfumlauf über die Leitungen (4), (5) und (6) mittels der an (3) entstehenden Konvektionsströmung (2) von oben anströmt. (7) ist die Entnahmeleitung für einen Reinigungsstrom (etwas nicht umgesetzter Ether und Höhersieder). Über (8) wird der Kolonne Wasser zugeführt, das über (9) wieder entnommen wird; das über (9) entnommene Wasser enthält mindestens einen Teil des bei der Spaltung entstehenden Alkanols. Der zu spaltende Alkyl-tert.-alkylether wird über die Leitung (10) der Kolonne zugeführt. Am Kolonnenkopf wird über (11) das bei der Spaltung entstehende tert.-Olefin entnommen.

Das über (11) entnommene tert.-Olefin wird in (12) kondensiert und teilweise über (13) als Rücklauf auf die Kolonne gegeben, teilweise über (14) als Produkt entnommen. Für den Fall, daß das tert.-Olefin als Azeotrop mit Alkanol über (11) entnommen werden soll, insbesondere bei Fortfall des oben beschriebenen Waschwasserstroms, kann der in der Zeichnung durch eine gestrichelte Linie umfaßte Bereich mit (12) und (13) entfallen; (11) mündet sodann direkt in (14).

Beispiele

Allgemeine Versuchsbeschreibung

Die Versuche wurden in einer Druckkolonne aus Stahl mit einem Innendurchmesser von 50 mm durchgeführt. Auf den Verdampfer waren drei Kolonnenschüsse zu je einem Meter montiert. Die Kolonne hatte 20 praktische Böden und war bis zu einem Betriebsdruck von 20 bar ausgelegt. Der Aufbau entsprach der beiligenden Zeichnung.

Alle in und aus der Kolonne führenden Produktströme waren mengengeregelt.

| Einsätze | | |
|---|---|---|
| Ether | Einspeisung | am Sumpf der Kolonne über (10) |
| Wasser | Einspeisung | am 18. Boden der Kolonne über (8) |
| Abnahmen | | |
| i-Amylen | Abzug | am Kopf der Kolonne über (11) |
| i-Buten | Abzug | am Kopf der Kolonne über (11) |
| Methanol | Abzug | am 10. Boden der Kolonne über (9) |
| Methanol/Wasser | Abzug | am 10. Boden der Kolonne über (9) |

Die Beheizung des Verdampfers erfolgte elektrisch.

Als Katalysator zur Etherspaltung wurde in einem Bett im Umlaufverdampfer handelsüblicher starksaurer, makroporöser Styrol-Divinylbenzol-Kationenaustauscher mit $SO_3H$-Gruppen (z.B. SPC 118 von Fa. Bayer) eingesetzt.

Die Ether wurden in einer Reinheit von > 99% eingesetzt.

Als Waschwasser wurde vollentsalztes Wasser eingesetzt.

Beispiel 1 Alleinige TAME-Spaltung in der Kolonne

Reaktionsbedingungen:

- Einsatz-TAME (gaschromatographisch) > 99,9 Gew.-%

- Sumpftemperatur ( = Spalttemperatur)      70,5°C
- Kopftemperatur      32,5°C
- Kolonnendruck      1,0 bar
- Rückflußverhältnis (R/E)      4,0
- Katalysatormenge      0,1 Liter
- zugesetzter TAME pro Stunde      0,6 Liter

Stoffe:

| Stelle | Einsatz | Abnahme | | | |
|---|---|---|---|---|---|
| | Einspeisung | Kopf | Seite | Sumpf | Summe |
| Komponente: | | | | | |
| Gesamt (g/h) | 438,0 | 247,9 | 167,1 | 23,0 | 438,0 |
| TAME (g/h) | 438,0 | < 0,1 | 77,3 | 20,3 | 97,6 |
| | | | | | |
| i-Amylen (g/h) | < 0,1 | 221,4 | 9,9 | 1,7 | 233,0 |
| Methanol (g/h) | < 0,1 | 26,5 | 79,9 | 0,7 | 107,1 |
| Höhere (g/h) | < 0,1 | < 0,1 | < 0,1 | 0,3 | 0,3 |

Umsatz TAME : 438,0 - 97,6 = 340,4 g (3,34 Mol)
= 77,7 %

Ausbeute i-Amylen : = 233,3 g (3,33 Mol)
Ausbeute Methanol : = 107,1 g (3,34 Mol)

Beispiel 2 TAME-Spaltung und Methanolextraktion in einer Kolonne

Reaktionsbedingungen:

- Einsatz-TAME (gaschromatographisch)    > 99,9 Gew.-%
- Sumpftemperatur ( = Spalttemperatur)    68,4 ° C
- Kopftemperatur    35,8 ° C
- Kolonnendruck    1,0 bar
- Rückflußverhältnis (R/E)    4,0
- Katalysatormenge    0,1 Liter
- LHSV (liquid hourly space velocity)    6,0

Stoffe:

| Stelle | Einsatz | Abnahme | | | |
|---|---|---|---|---|---|
| | Einspeisung | Kopf | Seite | Sumpf | Summe |
| Komponente: | | | | | |
| Gesamt (g/h) | 1038,0 | 284,0 | 731,0 | 23,0 | 1038,0 |
| TAME (g/h) | 438,0 | < 0,1 | < 0,1 | 15,8 | 15,8 |
| Wasser (g/h) | 600,0 | < 0,1 | 600,0 | 0,1 | 600,1 |
| i-Amylen (g/h) | < 0,1 | 284,0 | < 0,1 | 2,3 | 286,3 |
| Methanol (g/h) | < 0,1 | < 0,1 | 131,0 | 1,1 | 132,1 |
| Höhere (g/h) | < 0,1 | < 0,1 | < 0,1 | 3,7 | 3,7 |

Umsatz TAME : 438,0 - 15,8 = 422,2 g (4,14 Mol) = 96,3 %

Ausbeute i-Amylen : = 290,0 g (4,14 Mol)
Ausbeute Methanol : = 132,1 g (4,13 Mol)

Beispiel 3 MTBE-Spaltung und Methanolextraktion in einer Kolonne

Reaktionsbedingungen:

9

- Einsatz-MTBE (gaschromatographisch)     > 99,9 Gew.-%
- Sumpftemperatur ( = Spalttemperatur)     96,5 ° C
- Kopftemperatur     41,6 ° C
- Kolonnendruck     5,0 bar
- Rückflußverhältnis (R/E)     6,5
- Katalysatormenge     0,5 Liter
- LHSV (liquid hourly space velocity)     8,0

Stoffe:

| Stelle | Einsatz | Abnahme | | | |
|---|---|---|---|---|---|
| | Einspeisung | Kopf | Seite | Sumpf | Summe |
| Komponente: | | | | | |
| Gesamt (g/h) | 6960,0 | 1700,0 | 4970,0 | 290,0 | 6960,0 |
| MTBE (g/h) | 2960,0 | < 0,1 | 46,0 | 237,0 | 283,0 |
| Wasser (g/h) | 4000,0 | < 0,1 | 4000,0 | 0,1 | 4000,0 |
| i-Buten (g/h) | < 0,1 | 1700,0 | < 0,1 | 4,0 | 1704,0 |
| Methanol (g/h) | < 0,1 | < 0,1 | 924,0 | 49,0 | 973,0 |

$$\text{Umsatz MTBE:} \quad 2960,0 - 290,0 = 2670,0 \text{ g} \ (30,4 \text{ Mol})$$
$$= 90,2 \ \%$$

$$\text{Ausbeute i-Buten} = 1704,0 \text{ g} \ (30,4 \text{ Mol})$$
$$\text{Ausbeute Methanol} = 973,0 \text{ g} \ (30,4 \text{ Mol})$$

## Patentansprüche

1. Verfahren zur Spaltung von Alkyl-tert.-alkylethern in die zugrundeliegenden Alkanole und tert.-Olefine in Gegenwart von stark sauren Katalysatoren in einer Kolonnenapparatur, dadurch gekennzeichnet, daß der stark saure Katalysator unlöslich und hochpolymer, sowie anorganisch oder organisch ist und im Umlauf der Sumpfheizung der Kolonne bereitgestellt wird.

2. Verfahren nach Anspruch 1, dadurch gekennzeichnet, daß stark saure Kationenaustauscher in der $H^+$-Form eingesetzt werden.

3. Verfahren nach Anspruch 1, dadurch gekennzeichnet, daß der unlösliche, hochpolymere, stark saure Katalysator im Umlauf der Sumpfheizung der Kolonne von oben angeströmt wird.

4. Verfahren nach Anspruch 1, dadurch gekennzeichnet, daß der Sumpfumlauf eine Temperatur von 50 bis 200°C hat.

5. Verfahren nach Anspruch 4, dadurch gekennzeichnet, daß der Sumpfumlauf die aufgrund der Kolonnenbedingungen sich ergebende Siedetemperatur hat.

6. Verfahren nach Anspruch 2, dadurch gekennzeichnet, daß der den stark sauren Kationenaustauscher in der $H^+$-Form anströmende Sumpfumlauf eine Temperatur von 50 bis 100°C, bevorzugt 55 bis 80°C, besonders bevorzugt 60 bis 80°C hat.

7. Verfahren nach Anspruch 1, dadurch gekennzeichnet, daß am unlöslichen, hochpolymeren, stark sauren Katalysator eine LHSV (Liquid Hourly Space Velocity) von a = 1-100 Liter Sumpfumlauf pro Liter unlöslicher, hochpolymerer, stark saurer Katalysator pro Stunde, bevorzugt a = 10 bis 50, eingestellt wird.

8. Verfahren nach Anspruch 1, dadurch gekennzeichnet, daß dem als Kopfstrom der Kolonne entnommenen tert.-Olefin unterhalb der oberen Kolonnenböden ein Wasserstrom entgegengeschickt wird, der oberhalb des Kolonnensumpfes wieder der Kolonne entnommen wird und der 2 bis 6 Gew.-Teile pro 1 Gew.-Teil der auszuwaschenden Alkanolmenge beträgt.

## Claims

1. Process for the cleavage of alkyl tert-alkyl ethers into the underlying alkanols and tert-olefins in the presence of strongly acidic catalysts in a column apparatus, characterized in that the strongly acidic catalyst is insoluble and highly polymeric, and is inorganic or organic and is prepared in the circulation of the bottom heating of the column.

2. Process according to Claim 1, characterized in that strongly acidic cation exchangers in the $H^+$ form are employed.

3. Process according to Claim 1, characterized in that the insoluble, highly polymeric, strongly acidic catalyst is flowed onto from above in the circulation of the bottom heating of the column.

4. Process according to Claim 1, characterized in that the bottom circulation has a temperature of 50 to 200°C.

5. Process according to Claim 4, characterized in that the bottom circulation has the boiling temperature resulting owing to the column conditions.

6. Process according to Claim 2, characterized in that the bottom circulation flowing onto the strongly acidic cation exchanger in the $H^+$ form has a temperature of 50 to 100°C, preferably 55 to 80°C, particularly preferably 60 to 80°C.

7. Process according to Claim 1, characterized in that in the insoluble, highly polymeric, strongly acidic catalyst an LHSV (liquid hourly space velocity) of a = 1-100 litres of bottom circulation per litre of insoluble, highly polymeric, strongly acidic catalyst per hour, preferably a = 10 to 50, is set.

8. Process according to Claim 1, characterised in that a stream of water is sent to meet the tert-olefin taken from the column as the top stream below the upper column bottom, which stream of water is

EP 0 302 336 B1

taken from the column again above the column bottom and which is 2 to 6 parts by weight per 1 part by weight of the amount of alkanol to be washed out.

## Revendications

1. Procédé pour scinder des éthers d'alkyle et de tertio-alkyle en les alcanols et les tertio-oléfines de base en présence de catalyseurs fortement acides dans un appareil à colonne, caractérisé en ce que le catalyseur fortement acide est insoluble et hautement polymérisé ainsi qu'inorganique ou organique et est maintenu en place dans la circulation de chauffage du bas de la colonne.

2. Procédé suivant la revendication 1, caractérisé en ce qu'on utilise des échangeurs cationiques fortement acides sous la forme $H^+$.

3. Procédé suivant la revendication 1, caractérisé en ce que le catalyseur haut-polymère insoluble fortement acide est abordé par le haut par le courant circulant de chauffage du bas de la colonne.

4. Procédé suivant la revendication 1, caractérisé en ce que le courant circulant au bas de la colonne est à une température de 50 à 200°C.

5. Procédé suivant la revendication 4, caractérisé en ce que le courant circulant au bas de la colonne est à la température d'ébullition qui s'établit sur la base des conditions de la colonne.

6. Procédé suivant la revendication 2, caractérisé en ce que l'échangeur cationique fortement acide dans le courant circulant au bas de la colonne sous la forme $H^+$ est à une température de 50 à 100°C, de préférence de 55 à 80°C, notamment de 60 à 80°C.

7. Procédé suivant la revendication 1, caractérisé en ce qu'on règle au niveau du catalyseur haut-polymère insoluble fortement acide une VSHL (Liquid Hourly Space Velocity) a = 1-100 litres de courant circulant au bas de la colonne par litre de catalyseur haut-polymère insoluble fortement acide par heure, de préférence a = 10 à 50.

8. Procédé suivant la revendication 1, caractérisé en ce qu'on fait passer à contre-courant avec l'oléfine tertiaire prélevée comme courant de tête de la colonne au-dessous des plateaux supérieurs de cette dernière un courant d'eau qui est prélevé à nouveau au-dessus de la base de la colonne et qui représente 2 à 6 parties en poids par partie en poids de la quantité d'alcanol à extraire par lavage.

13

FIG.1